# EUROPEAN PATENT SPECIFICATION

(11) **EP 2 134 287 B1**
(45) Date of publication and mention of the grant of the patent: **20.05.2015**
(21) Application number: 08718238.2
(22) Date of filing: 26.03.2008
(51) Int. Cl.: A61B 17/86, A61B 17/16, A61B 17/34, A61B 19/00, A61C 1/08, A61B 17/17

(54) **A SURGICAL BONE MILLING INSTRUMENT**
CHIRURGISCHES KNOCHENFRÄSINSTRUMENT
INSTRUMENT DE TYPE FRAISE CHIRURGICALE À OS

(30) Priority: 12.04.2007 IT RE20070053
(43) Date of publication of application: 23.12.2009
(73) Proprietor: C.G.M. S.P.A., 42015 Correggio (Reggio Emilia) (IT)
(72) Inventor: PARMIGIANI, Corrado Saverio, 42015 Correggio (Reggio Emilia) (IT)
(74) Representative: Corradini, Corrado
(86) International application number: PCT/EP2008/053574
(87) International publication number: WO 2008/125449

(56) References cited:
- US-A- 4 681 103
- US-A- 5 989 025
- US-A- 5 997 515
- US-A1- 2004 142 300
- US-A1- 2005 222 575

## Description

### Technical Field

The present invention is a surgical bone milling instrument.

### Background Art

In general, the purpose of the instrument is the removal of areas of bone in a controlled manner in proximity to delicate parts of bone, typically to complete holes in bones in which the a terminal portion of the hole is located close to particularly delicate organs. A related prior art is shown in US2005/022575.

A typical application is in the surgical technique of mini-lifting of the maxillary sinus cavity, which involves the raising of the floor and filling with biomaterial of the maxillary cavity through a hole prepared in the bone for the insertion of a dental implant.

The cavity in the maxillary sinus is the largest of the pneumatic cavities in the cranial bone and it is located in the rear areas above the upper jaw.

The formation of the floor of the maxillary sinus can be influenced by the presence of the roots of premolar and molar teeth. At the apex of these roots there is a thin layer of compact cortical bone (maxillary sinus floor). The maxillary sinus cavity is normally unitary and contains air. The inside of the cavity is coated entirely with a mucus membrane known as the Schneider membrane.

The insertion of dental implants in the rear areas of the upper jaw is almost always conditioned by the presence of the maxillary sinus, which limits the availability of bone in height, especially in patients who have been edentulous for a long time.

Following the loss of the rear teeth of the jaw, a process of reabsorption of "external" bone begins, combined with a like reabsorption of the floor of the "internal" maxillary sinus, causing expansion of the maxillary sinus cavity and the approach of the sinus floor progressively closer to the "outside" edge of the alveolar crest, reducing the height of bone available for implantation.

In the 1990s the above-mentioned surgical techniques for the mini-lifting of the maxillary sinus were developed, substantially involving the boring of holes in the jaw for the insertion of dental implants and the raising of the floor of the maxillary sinus and filling with biomaterial of the maxillary cavity through the hole.

These techniques penetrates to the Schneider membrane, depending only on tactile sensation and radiographic investigations for its identification, since the membrane is not directly visible.

These techniques attracted the interest of dental surgeons less familiar with advanced surgery. The maxillary sinus mini-lifting technique offers functional advantages in view of the incidence of the operation.

The main difficulty of these techniques for raising the maxillary sinus is the creation of the final part of the bone cavity with decollement/detachment of the Schneider membrane from the "internal" bone surface in order to permit the filling of the sinus cavity with bone tissue (biomaterial). The limited thickness of the membrane or the slightest error of the operator can result in lacerations that compromise the success of the filling of the sinus cavity. The current techniques for mini-lifting can be divided on the basis of the instruments used, which are either osteotomes or mills.

Osteotomes are manual instruments, increasing in size and provided with a concave point with a cutting edge. Osteotomes can be used with depth limit stop rings adjusted manually with fixing screws. The stop is positioned according to the available bone height, established radiographically. An osteotome can be used with manual pressure or, in the case of hard bone, with the help of a surgical mallet. The special conformation of the osteotome means that once it is inserted into the implant hole, prepared to a distance of 1 to 2 mm from the floor of the maxillary sinus, it can remove a small quantity of bone tissue from the walls of the site and concentrate this on the terminal section of the osteotome. The bone cortex of the floor of the maxillary sinus is then fractured, by way of percussion with a mallet, to raise the floor of the sinus until the required lifting is achieved.

This operation requires extreme delicacy to avoid the possibility of lacerating the thin Schneider membrane.

In order to reduce the risk of laceration of the membrane, instead of using osteotomes alone, the insertion of biomaterial into the bone cavity has been proposed in order to act as padding to be compacted vertically between the osteotome point and the bone.

Mini-lifting mills are rotating instruments (500 rpm) for fitting on an electrical turbine and are fitted with a non cutting point and available in various calibrated lengths (one mill every millimetre) for sequential use. The mills act by wearing away the bone cortex that precedes the floor of the maxillary sinus, and because they are not sharp they can be used on the final portion of bone, limiting the risk of damage to the Schneider membrane.

After preparation of the site and after decollement and raising of the membrane using rounded manual instruments, the biomaterial is inserted before the positioning of the dental insert.

This technique again depends on the tactile sensitivity and practical experience of the operator.

One aim of the present invention is to realize a device capable of overcoming the difficulties mentioned above.

These and other aims are achieved by the invention as it is characterized in the accompanying claims.

### Disclosure of Invention

The invention is as claimed in claim 1.

The invention makes it possible to conduct axial milling of the bone cavity maintaining control of the position of the device relative to the cavity.

The instrument enables the operator to perceive the point at which the forward end reaches the end of the previously formed hole, or the end of the excavation created by the milling head. Furthermore, in each phase in which the milling element is rotated while the instrument is axially stationary inside the hole, a groove is created on the bottom of the hole (or the entire hole is elongated) the depth of which is constant and predetermined.

Furthermore, the operations of additional axial advance of the milling head can be checked with a probe element (when this is present).

Furthermore, by way of axial pressure applied to the milling head by the drive element (or applied to the probe element), the detachment of the residual portion of the bone wall can be achieved as soon as this has reached a breaking resistance lower than the force applied to the drive element (or to the probe element).

In particular, it can be foreseen that this detachment is achieved before the raised cutting edges of the milling head come into contact with the delicate Schneider membrane.

Furthermore, since the axial penetration of the instrument along the bone cavity is directly dependent on the angle of rotation of the same, the degree of axial penetration of the instrument, and thus the forward end thereof in the hole, can be kept under control.

### Brief description of the Drawings.

The invention is described in detail below with the support of the attached figures which illustrate a non-exclusive example embodiment of the same.
Figure 1 is a side view of a first embodiment of the instrument of the invention.
Figure 2 is a cross-section along the axial plane II-II of figure 1.
Figures 2A and 2B show the milling head 11 of figure 2, in enlarged scale, respectively in the advanced and withdrawn positions.
Figure 3 is a perspective view of figure 1.
Figures 4A and 4B show an enlarged detail of figure 1 in two different operating positions.
Figure 5 is the cross-section along the transverse plane V-V of figure 4 A.
figures from 6A to 6F show a sequence of operating stages in which the instrument excavates the final section of a hole in a bone.
Figure 7 is an exploded view of the instrument of figure 1.
Figure 8 is an axial cross-section of a second embodiment of the instrument of the invention.
Figure 9 is an axial cross-section as in figure 8, of a variant of the second embodiment.
Figure 10 is a perspective view of the forward end of the instrument of Figure 9.

### Best Mode for Carrying Out the Invention

The instrument 1 illustrated in figures from 1 to 5 comprises a milling element 10, in particular, with an elongate body shape, stretching along a longitudinal axis A, the forward end portion of which can rotate around the axis A and acts to mill bone. In particular, the forward end portion of the milling device 10 forms a milling head 11 which is equipped with sharp raised milling ridges 12, capable of milling bone in an axial direction when rotating.

The instrument 1 comprises a screw-threaded tubular element 30 through which the milling element 10 is inserted coaxially, with the milling head 11 thereof projecting beyond the end of the tubular element 30. In particular the tubular element 30 has a cylindrical tubular body, the outside surface of which has a circular cross-section and exhibits a thread 31a, which engages by helical coupling to the hole formed in the bone. The milling element 10 is associated to the tubular element 30, rotatably about the longitudinal axis A in relation to the tubular element 30, and axially translatably thereto.

The milling element 10 has a rear portion which coaxially passes through the tubular element; in particular, this comprises a tubular rod 14 of constant cross-section located behind the milling head 11, the external cylindrical surface of which couples with and matches the size of the internal cylindrical surface 32 of the tubular element 30, the length of the rod being greater than that of the tubular element 30. The head 11 has a greater external diameter than the rod 14 and the rear portion of the head 11 has a radial rear edge 11 b designed to stop in contact with the circular edge of the front end 30a of the tubular element 30. The external diameter of the head 11 is approximately equal to the external diameter of the tubular element 30; in particular, it is smaller than the external diameter of the thread 31 a.

The milling device 10 is solidly fixed to a drive element 40, located coaxially at the rear of the tubular element 30, driving the rotation of the milling element and to translate it axially relative to the tubular element 30. In particular, the drive element 40 is coaxially and solidly joined to the rear portion of the rod 14; the drive element 40 has a forward shank 41, of lower diameter, and a rear portion 42, of greater diameter, shaped as a circular handle for the manual activation of the milling element 10 in rotation and with axial translation. The rear portion 42 also serves as a handle for the manipulation of the entire instrument 1.

The instrument comprises means for limiting the axial translation of the milling element 10, combined with the drive element 40, relative to the tubular element 30.

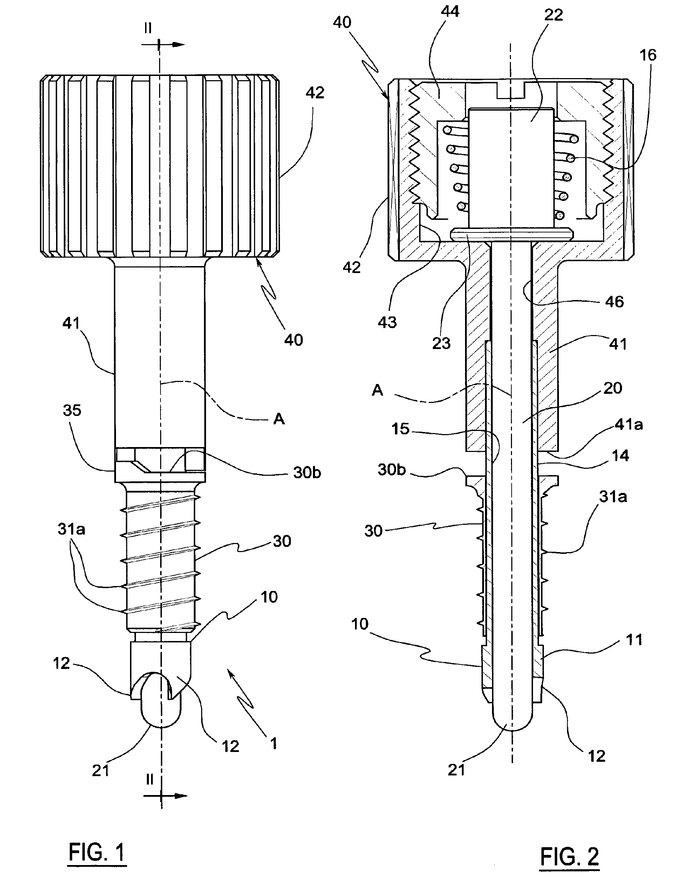

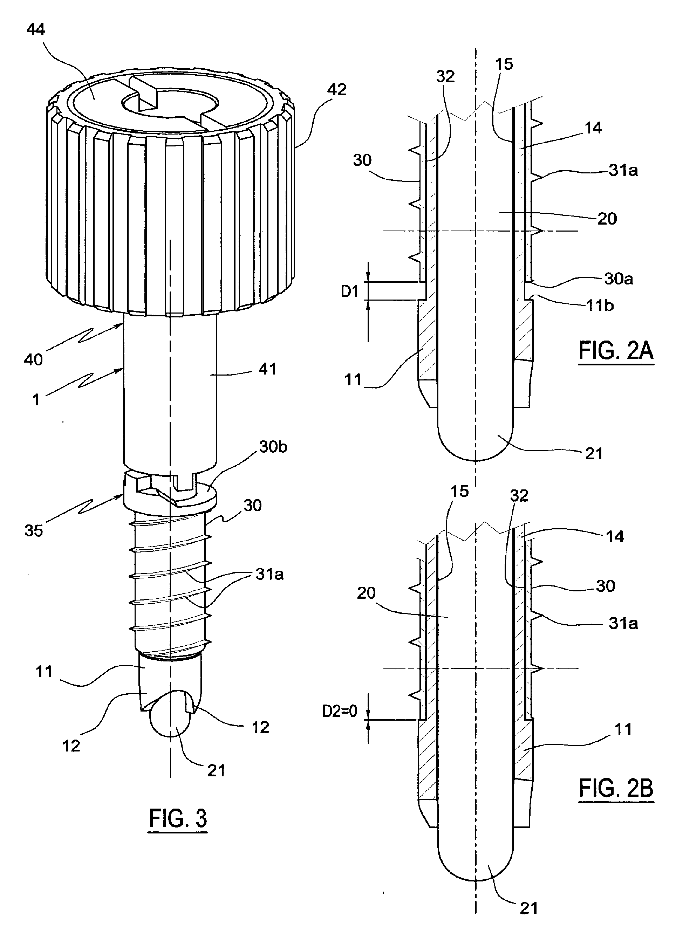

In the embodiment shown in figures 1 to 7, these means are determined by the length of the free axial portion of the rod 14 on which the tubular element can slide 30, relative to the length of the tubular element 30. The milling element 10 is free to move axially relative to the tubular element 30 between a fully *advanced position,* in which the milling head 11 projects forward the maximum possible distance D1 from the tubular element 30 (see figures 1, 2, and 2A), and a fully *withdrawn position,* in which the milling head 11 is the minimum possible distance D2 from the tubular element 30, in particular it is in contact with the same (D2 equal to zero) (see Figure 2B).

In the embodiment shown in the figures, the drive element 40 transmits torque to the head 11 to which it is solidly constrained, and also to the tubular element 30 in order to produce the rotation of the same.

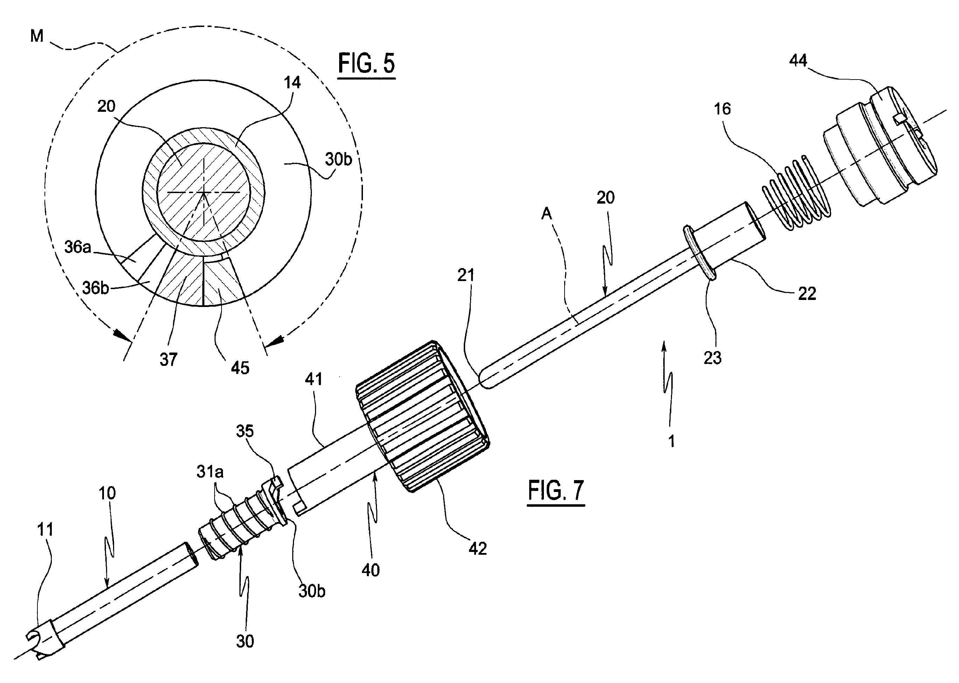

In particular, the drive element 40, is free to slide axially, over a certain distance, relative to the tubular element 30, and to rotate, again relative to the tubular element 30, and is torsionally engaged there-with by mutual engagement means that leave the drive element 40 free to be rotated by an angle M (figure 5) of less than 360 degrees and to be axially shifted in relation to the tubular element 30.

In particular, the means of reciprocal engagement comprise profiled raised portions 35 and 45 extending in an axial direction towards each other (one faces back and the other forward), from the rear end circular edge 30b of the tubular element 30 and respectively from the forward end circular edge 42a of the shank 42 of the drive element, the raised portions of which are designed to reciprocally contact following reciprocal rotation in order to transmit torque.

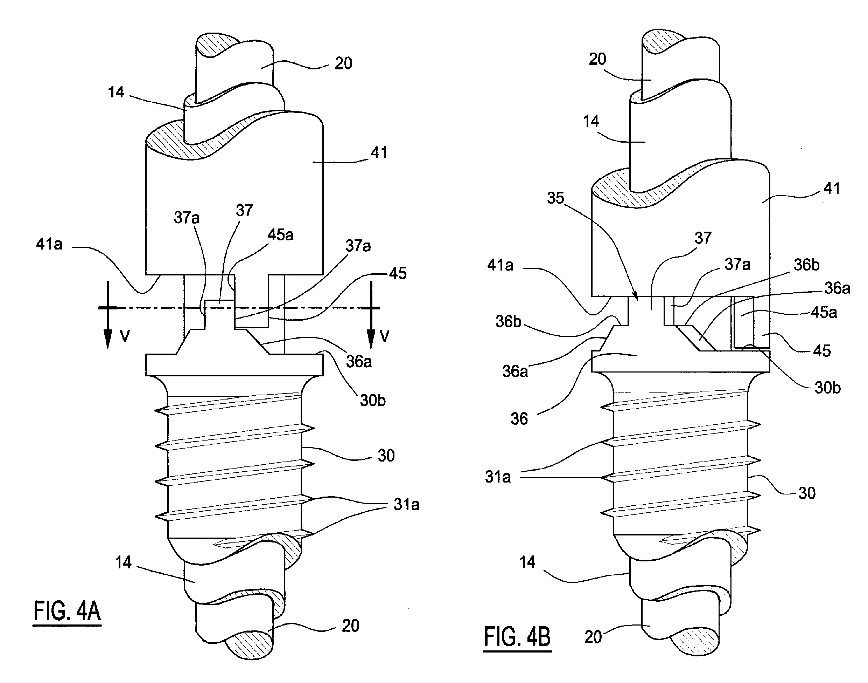

In particular, the profiled raised portions 35 and 45 are shaped so that, when they are in reciprocal torsional contact (figure 4A), they maintain the tubular element 30 and the drive element 40 at a predefined maximum axial distance (which substantially corresponds to the above-mentioned *fully withdrawn position* of the milling element 10); furthermore, they enable the approach of the two elements 30 and 40 until a minimum axial distance is reached (which substantially corresponds to the above-mentioned *fully advanced position* of the milling element 10) by angular translation relative to the reciprocal torsional contact position (figure 4B).

In detail, the profiled raised portion 35, united to the tubular element 30, has a widened base 36 with two surfaces 36a which are inclined and converging, and a central raised straight portion 37 with two parallel sides of axial development 37a that project axially relative to the base 36. The other raised portion 45, united to the drive element 40, is shaped as a raised central portion with two parallel sides of axial development 45a (figures 4A, 4B). Alternatively, the raised portion together with the tubular element 30 can have the shape described herein for the raised portion joined to the drive element 40 and vice-versa.

Rotating the drive element 40 relative to the tubular element 30, the raised portion 45 first comes into contact with the base 36 of the relief portion 35, then slides along one of the inclined surfaces 36a and then, as it approaches the central raised portion 37, the two elements 30 and 40 move away from each other axially. When the axial sides 37a and 45a then come into reciprocal contact, the tubular element 30 and the drive element 40 are situated at a predefined distance (corresponding to the fully withdrawn position of the milling head 11), without the possibility of moving closer, due also to the fact that at the base of the central raised portion 37 two sections 36b are located on transverse planes (at 90 degrees relative to the axial direction) serving as end stops against the highest extremity of the raised portion 45, preventing its approach in an axial direction.

Rotating the drive element 40 relative to the tubular element 30, so as to distance the two relief portions 35 and 45 from each other, renders the rotary movement of the element 40 independent relative to element 30, up to an angle M less than 360 degrees (figure 5). Furthermore, these elements can be moved axially towards each other, until the upper apex of the relief portion 37 stops in contact with the edge 41 a of the shank 41 of element 40 (or the upper apex of the relief portion 45 stops in contact with the edge 30b of element 30), which determines the minimum distance between elements 30 and 40.

In a preferred embodiment, shown in figures 1-5, the instrument 1 comprises a probe element 20 of elongated form which passes internally and coaxially through the milling element 10 and slides longitudinally, along axis A, through the same, with its forward end 21 designed to project relative to the forward end of the milling element 10. In particular, the probe element 20 is inserted into an axial through-hole 15 formed in the milling element 10; the raised milling ridges 12 thus have, in this case, a radial extension limited by the presence of the hole 15 in the head 11.

In particular, the probe element 20, at least in its forward and mid portions, has the form of a cylindrical rod, possibly with the forward end 21 rounded in order not to harm the tissues with which it comes into contact.

The milling element 10 affords a cylindrical axial through-hole 15, which is followed by a coaxial hole 46 of substantially the same diameter, which continues through the drive element 40; the probe device 20 is snugly fitted inside the holes 15 and 46, the probe device 20 being subject to the action of elements pushing it axially so that the forward end 21 thereof projects beyond the forward end of the milling element.

The probe element 20 signals to the operator, possibly visually, when the milling head 11 reaches the end of the hole into which it is inserted during the use of the instrument and, from that point onwards, signals the position of the milling head 11 in the subsequent operations of further axial advance of the same.

Further, by way of an axial pressure applied to the probe element 20, it is possible to cause the detachment of the residual portion of bone wall, when the bone wall has reached a resistance to breakage that is lower than the force applied to the probe element 20.

The probe element 20 has a rear portion 22 visible to the operator.

In particular, the rear portion 22 of the probe element is designed to be visible at the rear of the handle formed by the rear portion of the drive element 40.

The probe element 20 can be translated axially, a motion produced by pressure applied to its rear portion 22, causing the forward end 21 thereof to project by a predefined distance with respect to the forward end of the milling element 10.

In the embodiment shown in the figures, the rear portion 22 of the probe element 20 has a greater diameter than the axial cavity, thus forming a stop for forward axial movement thereof, and it is subject to the axial pressure of a precompressed helical spring 16 acting to push the probe element 20 forward.

In particular, the rear portion 22 is housed in a concavity 43 formed in the rear portion 42 coaxial with axis A and facing the rear; the spring 16 is located inside this concavity and is compressed between a radial raised portion 23 of the rear portion 22 and a stop element 44 coupled with the concavity 43 helically, thereby making it possible to adjust its axial position and thus the level of precompression of the spring 16, and consequently the axial pressure acting on the probe element 20.

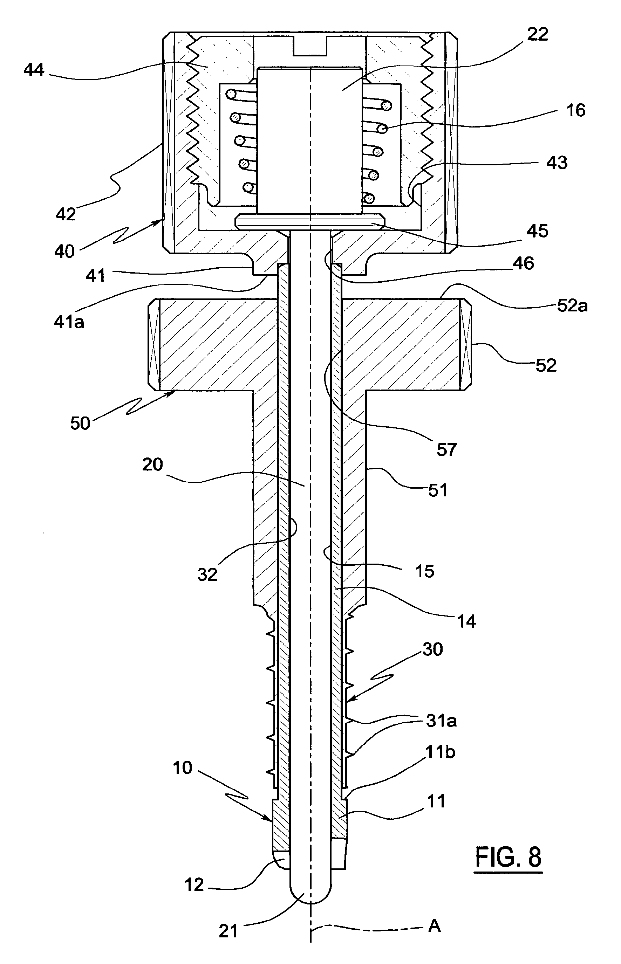

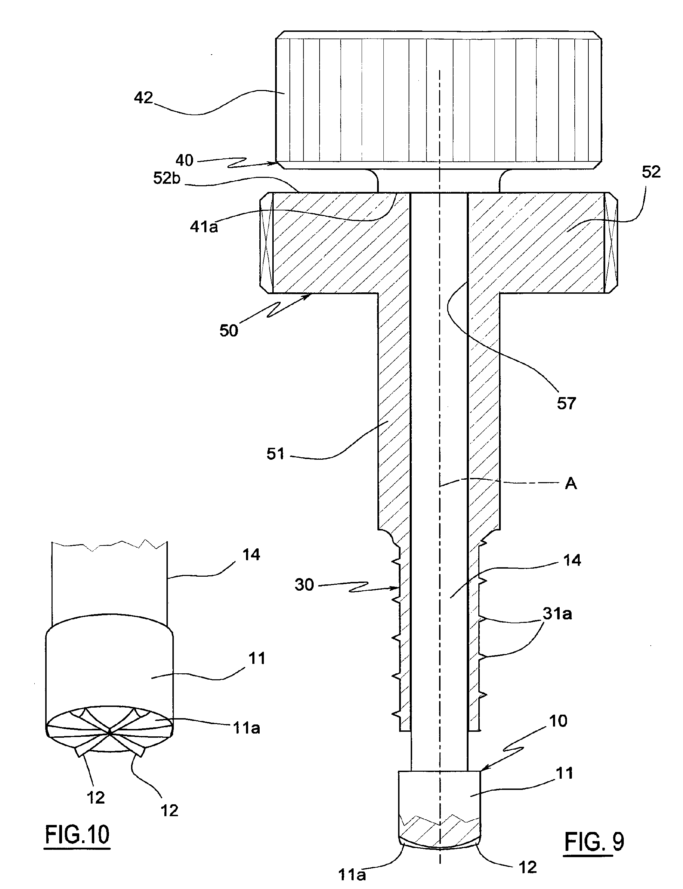

Figures 8 and 9 show a further embodiment of the invention.

This second embodiment differs from the first principally due to the fact that the drive element 40 rotates only the milling element 10 and not the tubular element 30 too; consequently the described profiled raised portions 35 and 45 are absent and the drive element 40 can rotate freely relative to the tubular element 30. The tubular element 30 is instead rotated by a dedicated second drive element 50, independent of the first drive element 40.

In particular the drive element 50 is located forward of the drive element 40 and is coaxially and solidly fixed to the rear end portion of the tubular element 30; the rod 14 of the milling element 10 passes through a matching axial through-hole 57 formed in the drive element 50.

The second drive element 50 comprises a tubular shank 51 fixed coaxially and solidly to the rear end portion of the tubular element 30 and a rear portion 52, of greater diameter relative to the shank 52, shaped as a circular handle that enables the operator to manually activate the milling element 10, with his fingers, in rotation and in axial translation. Preferably, the rear portion 52 has a greater diameter than the corresponding portion 42.

The two drive elements 40 and 50 are free to rotate reciprocally, even when in reciprocal contact.

This embodiment too includes elements acting to limit the axial movement of the milling element 10 relative to the tubular element 30 to a predetermined extent.

In particular, the milling element 10, together with the drive element 40, is free to move axially relative to the tubular element 30 and the second drive element 50, between a *maximum advanced position,* in which the milling head 11 projects forward the maximum possible distance D1 from the tubular element 30, and a *maximum withdrawn position,* in which the milling element 11 is at the minimum possible distance D2 from the tubular element 30, in particular, in contact there-with (D2 equal to zero).

The axial movement between the elements 10 and 30 is delimited between the contact of a forward end edge 30a of the tubular element 30 with a rear end edge 11b of the milling head 11 (which determines the *maximum advanced position*) (figure 2A) and the contact of a transverse rear surface 52b of the rear portion 52 with a forward circular protrusion 42a of the shank 42 (which determines the *maximum withdrawn position*) (figure 2B).

In the version of the second embodiment shown in figure 8, the instrument has a probe element 20 with the characteristics described above for the first embodiment of the invention.

In the version shown in figures 9 and 10, the instrument 1 does not have the described probe element 20. Consequently, the milling element 10 is not axially hollow and the milling head 11 has a continuous forward frontal surface 11 a. In this case the raised milling ridges 12 can extend radially over the entire surface 11 a (see figure 10).

There follows an example of use of the instrument.

A typical use of the instrument illustrated is to extend the final part of an initially blind hole, realized previously in a jaw bone.

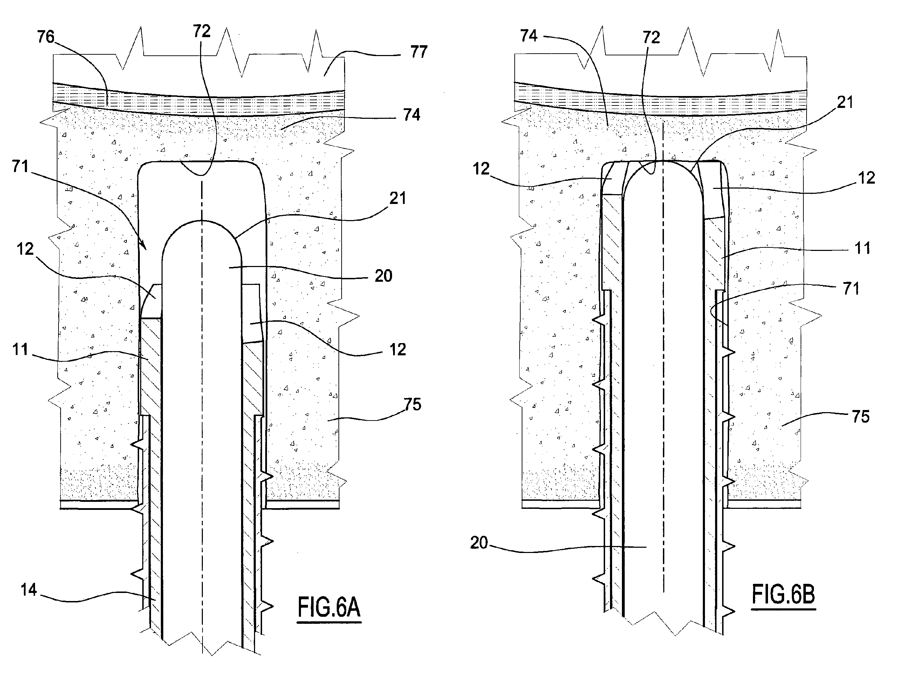

Figure 6A shows the preliminary blind section 71 of the hole, which is formed in the jaw bone 75 according to the known art (preferably using a motorized milling instrument, possibly with the use of radiographic viewing methods), the end surface 72 of which lies at a relatively short, though safe, distance (a few millimetres) from the Schneider membrane 76 located on the bottom of the maxillary sinus 77.

The instrument 1 is suitable for extending the final part of the hole 71 until breaking or destroying the thin upper wall 74 of bone cortex which separates the end surface 72 of the hole 71 from the membrane 76, without causing harmful lesions to the membrane 76.

For this purpose, first (figure 6A), the tubular element 30 is rotated inside the hole 71, such that the helical thread 31 a engages with the lateral cylindrical surface of the hole 71, into which the sharp-edged thread penetrates. At this point the milling head 11 is in the above-mentioned *withdrawn position* relative to the tubular element 30 (in particular, they are in contact).

In a first phase (advancement phase), by rotating the element 30 the penetration of the forward end of the instrument 1 is induced (the milling head 11 and the forward part of the tubular element 30) inside the hole 71. The instrument is made to advance axially in the hole until the forward end of the milling head 11 comes into contact with the end surface 72 (Figure 6B). The point at which the milling head 11 comes into contact with the end surface 72 is perceived, both because the operator notices an increase in the resistance to rotation of the tubular element 30, and because they perceive that the milling head 11 is axially locked in the above-mentioned *withdrawn position,* without the possibility of being moved axially forward.

Further, if the instrument includes the probe element 20, its forward end 21 stops against the surface 72, until it is aligned with the forward end of the milling head 11 (figure 6B); the rear portion 22 of the probe 20, is pushed back inside the hole 15 and moves to a position, relative to the rear portion of the drive element 40, which is perceived visually by the operator and can be used as an initial reference position.

In the first embodiment of the instrument, the rotation of the tubular element 30 is produced by manually rotating the drive element 40, which transmits, through the reciprocally engaging elements 35 and 45, a torque to the tubular element 30.

In the second embodiment of the instrument, the rotation of the tubular element 30 is produced by manually rotating the drive element 50, which drives only the element 30; the milling head 11 is not set in rotation.

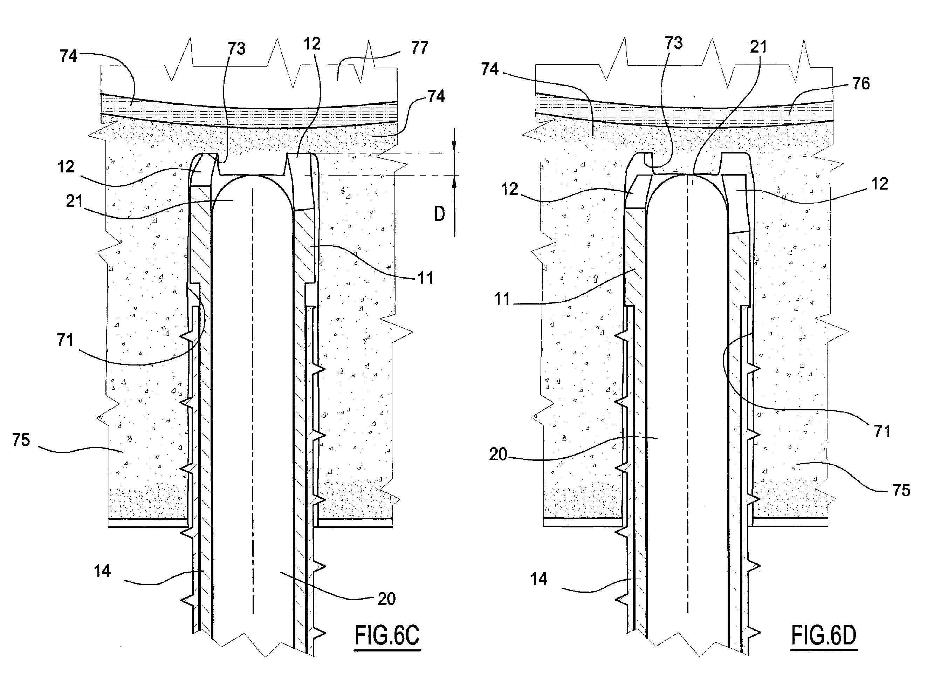

In the second phase (milling phase), only the milling element 10 is rotated and it is moved axially forward relative to the tubular element 30 to excavate, in the axial direction, a cavity on the bottom of the hole 71 (see figure 6B). If the raised milling ridges 12 have a limited radial extension, for example due to the presence of the hole 15 in the head 11 (figures 1-8), the cavity that they create has the form of a peripheral circular groove 73 with a maximum axial depth of D (figure 6C), which develops along the circumference of the surface 72 delimiting a circular central portion.

In the first embodiment, operation is manual using the drive element 40: the raised portion 45 is distanced from the raised portion 35, and the milling element 10 is rotated relative to the tubular element 30, with alternating rotation by an angle included within the above mentioned angular range M which does not engage with the raised portion 35 (figure 5). At the same time the milling head 11 is pushed forward manually so that the combined actions (axial pressure and rotation) cause the removal of bone tissue at the point of contact of the raised milling ridges 12.

In the second embodiment, operation is manual using the drive element 50 on the milling head 11, rotating it and at the same time pushing it axially forward so that the combined actions (axial pressure and rotation) cause the removal of bone tissue at the point of contact of the raised milling ridges 12. This milling action continues at most until the milling head 11 reaches the maximum advanced position, this being to a depth equal to the difference D between the maximum distance D1 and the minimum distance D2. Consequently, a peripheral circular groove 73 is created on the bottom surface 72 with an axial depth of maximum depth D, which develops around the circumference of the bottom surface 72, axially approaching the membrane 76.

In the subsequent phase (third phase, advancement), the tubular element 30 is further rotated such as to cause a further axial advance of the instrument proportional to the imposed rotation.

In particular, in the first embodiment, the tubular element 30 is newly rotated manually, using the drive element 40 in order to act on the tubular element 30 through the reciprocally engaging elements 35 and 45, causing a further axial advance of the element 30 proportional to the imposed rotation. In this phase, following the approach into contact of the raised portions 35 and 45 the milling element 10 is first returned to the fully withdrawn position relative to the tubular element 30, and its raised milling ridges 12 are withdrawn from the groove 73 (see figure 6D) so that, while the tubular element 30 rotates and screws into the hole 71, the milling ridges 12 do not mill the bone. Alternatively the rotation of the tubular element 30 is imposed by the drive element 50 (in the second embodiment) which acts directly on the tubular element 30; in this case the milling head 11 remains stationary and the raised milling ridges 12 do not mill the bone.

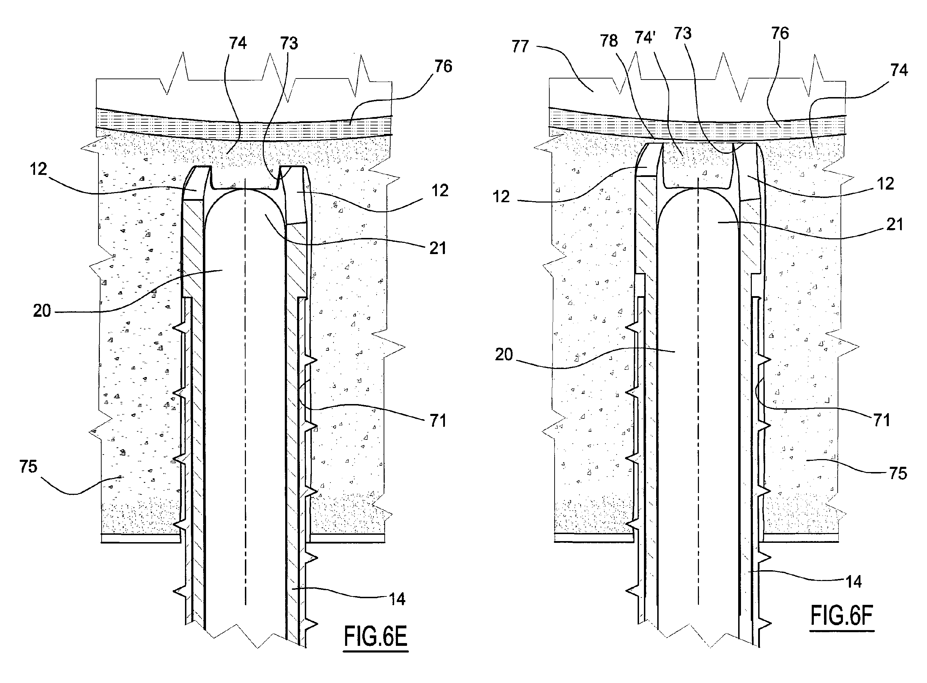

When the milling head 11 comes into contact with the end surface of the peripheral groove (see figure 6E), this is perceived in the way described above for the first operating phase, and the penetration of the tubular element 30 is thus stopped.

Furthermore, if the instrument includes the probe element 20, the operator perceives when the rear portion of the probe has moved by a certain measurable distance, relative to the initial reference position.

In the subsequent phase (fourth phase, milling) only the milling element 10 is rotated in order to increase by a further axial distance the depth of the circular groove 72 created during the previous phase (figure 6F). In practice, the milling element 10 is operated in the same way described above for the second phase.

It is possible to proceed with further cycles of advancing of the tubular element 30 and subsequent milling as described above, progressively increasing the depth of the groove 73 until the residual portion 74' of the wall 74 is defined, in the form of a small disk delimitated by the groove, remaining attached to the jaw bone 75 by a residual layer 78 of relatively very thin bone (see figure 6F).

In order to finally detach this residual portion 74', which closes the hole 71, from the bone 75, an axial pressure of sufficient force can be applied on the handle 42, the handle 42 transmitting this pressure to the milling head 11, to break the layer 78 and detach the residual portion 74'. This pressure can be produced manually by the operator, or by dedicated pushing devices (not shown in the figures), for example, a spring pushing the head 11 towards the maximum advanced position relative to the tubular element 30.

If the probe element 20 is present a predefined axial pressure can be applied to the probe element 20 sufficient to detach the residual portion 74'. This pressure can be produced by the spring 16 which automatically breaks the layer 78, detaches the portion, and opens the hole 71 towards the membrane 76; the membrane 76 is not damaged because the probe element 20 is designed with a rounded tip.

Alternatively, it can be arranged that the axial pressure on the probe element 20 is applied manually by the operator, who, for example, pushes with a finger on the rear portion of the probe element 20 which extends outside of the drive element 40.

Alternatively, when the residual portion of bone wall 74 is sufficiently weakened, the tubular element 30 can be rotated so that the milling head 11 is made to advance axially against the end surface until the residual portion 74 is detached.

If the instrument does not include the probe element 20 and the raised milling ridges 12 extend radially across the entire surface 11 a, the method for realizing the final section of a hole 71, formed previously using the instrument 1, involves the same operational phases described above, the sole difference being that the head 11 excavates a cavity in an axial direction, not in the form of a circular groove defining a disk-shaped residual portion 74'; the head 11 instead excavates a channel of circular cross-section which represents an axial elongation of the hole 71 and with substantially the same cross-section as the hole 71. The procedure involves advancing the hole 71 until this comes into contact with the membrane 76; or until the bone is reduced to a thin layer (of a few millimetres) that separates the hole 71 from the membrane 76, which can then be broken using the same methods described above in order to detach the residual portion 74'.

In a further alternative embodiment (not shown in the figures), the drive element 40 is mechanically rotated, for example by drive transmission elements equipped to the rear part of the same.

A similar mechanical drive solution can also be applied to the second drive element 50 of the second embodiment described above.

The invention makes it possible to conduct axial milling of the bone hole maintaining control of the position of the device relative to the hole.

The instrument 1 enables the operator to perceive, by way of the resistance encountered during axial advance, by screwing, along the hole 71, possibly also through the action of the probe 20, the point at which the milling head 11 reaches the end 72 of the previously-formed hole 71, or the bottom of the cavity produced by the milling head 11. Furthermore, every phase in which the milling element 10 is rotated while the tubular element 30 is stationary, produces a groove 73 (or an extension of the hole 71, in the case of a head with raised milling ridges 12 extending across the entire surface 11a) the depth of which has a constant predefined value; in particular, equal to the value (D1 - D2) of the axial translation completed by the head 11 passing from the maximum withdrawn position to the maximum advanced position. The perception of the point to which the advancement phase is conducted (the milling head 11 reaches the bottom of the previously formed hole 71, or the bottom of the cavity produced by it) and knowing the depth of the milling phase (the cavity realized by the milling head 11), it is possible to have constant knowledge and control of the depth of the cavity being created. Furthermore, the operations of further axial advancement of the milling head 11 can be checked with the probe element 20 (when this is present); in particular, while the milling element 10 is made to advance further, to excavate a further axial section of cavity, the probe element 20 remains axially stationary against the central area of the end surface 72 (which is not subjected to milling). Consequently, since the probe element 20 is designed to have millimetre markings, it becomes a means for constant control and for determining the measured position of the milling head 11 relative to the position of the end 72 of the bone hole.

Furthermore, by way of an axial pressure applied to the drive element 40 (or to the probe element 20) detachment can be achieved of the residual portion 74', of the bone wall, as soon as the latter has reached a degree of breaking resistance less than the force applied to the drive element 40 (or to the probe element 20).

In particular, it can be arranged so that this detachment occurs before the raised cutting ridges 12 have exceeded, even at a single point, the thickness of the wall 74; the detachment thus occurs without the cutting ridges being able to come into contact with the delicate Schneider membrane. Furthermore, since the penetration of the tubular element 30 along the axis of the hole 71 is directly dependent on the angle of rotation of the same, by way of the pitch of the thread 31 a, through the control of the rotation applied to the tubular element 30, it is possible to maintain control of the extent of axial penetration of the tubular element 30, and thus the forward end of the instrument, in the hole 71.

Obviously, as regards the present invention numerous modifications could be introduced of a practical-technical nature, without forsaking the range of the invention as claimed below.

## Claims

1. A surgical bone milling instrument, serving to operate in a hole formed in bone, comprising a milling element (10), having a longitudinal axis (A) and a forward end portion (11) capable of rotating around a longitudinal axis (A), and destined to mill a bone, **characterized in that** it comprises:
a tubular element (30) having an external surface with a circular cross-section and being provided with a thread destined to engage, by helical coupling, in the hole formed in the bone, the milling element (10) being inserted coaxially through the tubular element (30) so that the forward end portion (11) of the milling element (10) is located forward of the tubular element (30), and with the possibility of rotating around the longitudinal axis (A) relative to the tubular element (30) and the possibility of axial movement relative to the tubular element (30);
means for limiting to a predetermined extent the axial movement of the milling element (10) relative to the tubular element (30),
and further comprises a drive element (40), located rear of the tubular element (30), fixed to the milling element (10), rotating the milling element (10) and moving it axially in relation to the tubular element (30).

2. The instrument of claim 1 **characterized in that** the milling element (10) has a rear portion (14) that passes coaxially through the tubular element (30) while the forward end portion (11) projects forward of the tubular element (30).

3. The instrument of claim 1, **characterized in that** the drive element (40) acts to transmit torque to the tubular element (30).

4. The instrument of claim 3, **characterized in that** the drive element (40), is free to slide axially and rotate relative to the tubular element (30) and is torsionally engaged with the latter by means of reciprocal engagement (35, 45) which leave the drive element (40) free to be rotated by an angle of less than 360 degrees, and to be moved axially relative to the tubular element (30).

5. The instrument of claim 4, **characterized in that** the means of reciprocal engagement (35, 45) comprise profiled raised portions protruding in an axial direction from the tubular element (30) and respectively from the drive element (40), which are designed to come into reciprocal contact following reciprocal rotation in order to transmit a torque drive.

6. The instrument of claim 5, **characterized in that** the profiled raised portions (35, 45) are arranged in order to maintain at a predetermined maximum axial distance the tubular element (30) and the drive element (40) when these elements are positioned in reciprocal torsional contact, and to permit the approach to a minimum axial distance of the two elements by angular translation relative to the position of reciprocal torsional contact.

7. The instrument of claim 1, **characterized in that** it comprises a second drive element (50) fixed to the tubular element (30) and driving the tubular element (30) in rotation.

8. The instrument of claim 7, **characterized in that** the second drive element (50) is coaxial with, and located forward of, the first drive element (40).

9. The instrument of claim 1, **characterized in that** it comprises pushing elements acting to axially push the milling head (11) forward relative to the tubular element (30).

10. The instrument of claim 1, **characterized in that** it comprises a probe element (20) of elongate shape located internally of and coaxially to the milling element (10) and being longitudinally slideably through the milling element (10), with the forward end projecting relative to the forward end of the milling element (10).

11. The instrument of claim 10, **characterized in that** it comprises means for signalling movement of the probe element (20) relative to the milling element (10).

12. The instrument of claim 10, **characterized in that** it comprises means for axially pushing the probe element (20) in order to cause a forward end thereof to project beyond a forward end of the milling element (10).

13. The instrument of claim 10, **characterized in that** it comprises means for adjusting an axial pressure applied on the probe element (20).

14. The instrument of claim 10, **characterized in that** the probe element (20) exhibits a rear portion which is visible to the operator.

15. The instrument of claim 10, comprising a handle for manipulation of the instrument, **characterized in that** the rear portion of the probe element (20) is designed to remain visible at the rear of the handle.

16. The instrument of claim 15, **characterized in that** the probe element (20) moves axially by way of a pressure applied to its rear portion, causing the forward end thereof to project by a predefined distance beyond the end of the forward end of the milling element (10).

17. The instrument of claim 10, **characterized in that** the probe element (20) exhibits millimetric markings for constant control and determination of a measured position of the milling head (11) relative to the position of the end (72) of the bone hole.

## Patentansprüche

1. Chirurgisches Knochenfräsinstument, das dazu dient, in einer in einem Knochen gebildeten Öffnung zu operieren, umfassend ein Fräselement (10), das eine Längsachse (A) und einen vorderen Endabschnitt (11), der in der Lage ist, sich um eine Längsachse (A) zu drehen, aufweist und dazu bestimmt ist, einen Knochen zu fräsen, **dadurch gekennzeichnet, dass** es Folgendes umfasst:
ein röhrenförmiges Element (30) mit einer äußeren Oberfläche mit einem kreisrunden Querschnitt, das mit einem Gewinde ausgestattet ist, welches dafür bestimmt ist, durch Schraubenkopplung in Eingriff mit der in dem Knochen gebildeten Öffnung zu gelangen, wobei das Fräselement (10) koaxial durch das röhrenförmige Element (30) hindurch eingeführt wird, so dass sich der vordere Endabschnitt (11) des Fräselements (10) vorderhalb des röhrenförmigen Elements (30) befindet, und mit der Möglichkeit, sich in Bezug auf das röhrenförmige Element (30) um die Längsachse (A) zu drehen, und der Möglichkeit der axialen Bewegung in Bezug auf das röhrenförmige Element (30),
Mittel zum Begrenzen eines festgelegten Umfangs der axialen Bewegung des Fräselements (10) in Bezug auf das röhrenförmige Element (30),
und ferner ein Antriebselement (40) umfasst, das sich nahe dem röhrenförmigen Element (30) befindet, am Fräselement (10) befestigt ist und das Fräselement (10) dreht und es axial in Bezug auf das röhrenförmige Element (30) bewegt.

2. Instrument nach Anspruch 1, **dadurch gekennzeichnet, dass** das Fräselement (10) einen hinteren Abschnitt (14) aufweist, der koaxial durch das röhrenförmige Element (30) läuft, während der vordere Endabschnitt (11) vorderhalb des röhrenförmigen Elements (30) hervorsteht.

3. Instrument nach Anspruch 1, **dadurch gekennzeichnet, dass** das Antriebselement (40) zum Übertragen von Drehmoment auf das röhrenförmige Element (30) dient.

4. Instrument nach Anspruch 3, **dadurch gekennzeichnet, dass** das Antriebselement (40) frei axial gleiten und sich in Bezug auf das röhrenförmige Element (30) drehen kann und mit Letzterem über Mittel zum gegenseitigen Eingriff (35, 45) in Torsionseingriff steht, die das Antriebselement (40) frei um einen Winkel von weniger als 360 Grad drehen und axial in Bezug auf das röhrenförmige Element (30) drehen lassen.

5. Instrument nach Anspruch 4, **dadurch gekennzeichnet, dass** die Mittel zum gegenseitigen Eingriff (35, 45) profilierte erhöhte Abschnitte umfassen, die in einer axialen Richtung von dem röhrenförmigen Element (30) und jeweils von dem Antriebselement (40) hervorstehen und die dafür gestaltet sind, nach gegenseitiger Drehung in gegenseitigen Kontakt zu kommen, um eine Drehmomentantriebskraft zu übertragen.

6. Instrument nach Anspruch 5, **dadurch gekennzeichnet, dass** die profilierten erhöhten Abschnitte (35, 45) angeordnet sind, um das röhrenförmige Element (30) und das Antriebselement (40) in einem festgelegten maximalen axialen Abstand zu halten, wenn diese Elemente in gegenseitigem Torsionskontakt stehen, und um die Annäherung der beiden Elemente auf einen minimalen axialen Abstand durch schräge Translation in Bezug auf die Position des gegenseitigen Torsionskontaktes zu ermöglichen.

7. Instrument nach Anspruch 1, **dadurch gekennzeichnet, dass** es ein zweites Antriebselement (50) umfasst, das an dem röhrenförmigen Element (30) befestigt ist und das röhrenförmige Element (30) zur Drehung antreibt.

8. Instrument nach Anspruch 7, **dadurch gekennzeichnet, dass** das zweite Antriebselement (50) koaxial zum ersten Antriebselement (40) und vorderhalb desselben angeordnet ist.

9. Instrument nach Anspruch 1, **dadurch gekennzeichnet, dass** es Schiebeelemente umfasst, die dazu dienen, den Fräskopf (11) in Bezug auf das röhrenförmige Element (30) axial vorwärts zu treiben.

10. Instrument nach Anspruch 1, **dadurch gekennzeichnet, dass** es ein Sondenelement (20) von länglicher Form umfasst, das innerhalb des Fräselements (10) und koaxial zu diesem angeordnet ist und längs durch das Fräselement (10) gleitbar ist, wobei das vordere Ende in Bezug auf das vordere Ende des Fräselements (10) hervorsteht.

11. Instrument nach Anspruch 10, **dadurch gekennzeichnet, dass** es Mittel zum Signalisieren von Bewegung des Sondenelements (20) in Bezug auf das Fräselement (10) umfasst.

12. Instrument nach Anspruch 10, **dadurch gekennzeichnet, dass** es Mittel zum axialen Schieben des Sondenelements (20) umfasst, um zu bewirken, dass sein vorderes Ende über ein vorderes Ende des Fräselements (10) hinaus hervorsteht.

13. Instrument nach Anspruch 10, **dadurch gekennzeichnet, dass** es Mittel zum Justieren eines auf das Sondenelement (20) ausgeübten axialen Drucks umfasst.

14. Instrument nach Anspruch 10, **dadurch gekennzeichnet, dass** das Sondenelement (20) einen hinteren Abschnitt aufweist, der für den Bediener sichtbar ist.

15. Instrument nach Anspruch 10, einen Griff zum Manipulieren des Instruments umfassend, **dadurch gekennzeichnet, dass** der hintere Abschnitt des Sondenelements (20) dafür gestaltet ist, am Ende des Griffs sichtbar zu bleiben.

16. Instrument nach Anspruch 15, **dadurch gekennzeichnet, dass** sich das Sondenelement (20) aufgrund eines auf seinen hinteren Abschnitt ausgeübten Drucks axial bewegt, was bewirkt, dass sein vorderes Ende um eine vorab definierte Strecke über das Ende des vorderen Endes des Fräselements (10) hervorsteht.

17. Instrument nach Anspruch 10, **dadurch gekennzeichnet, dass** das Sondenelement (20) Millimetermarkierungen zur konstanten Kontrolle und Bestimmung einer abgemessenen Position des Fräskopfs (11) in Bezug auf das Ende (72) der Knochenöffnung aufweist.

## Revendications

1. Instrument chirurgical de fraisage des os servant à opérer dans un trou formé dans l'os, comprenant un élément de fraisage (10) présentant un axe longitudinal (A) et une partie terminale avant (11) capable de tourner autour de l'axe longitudinal (A), et destiné à fraiser un os, **caractérisé en ce qu'**il comprend :
un élément tubulaire (30) comportant une surface externe à section transversale circulaire et pourvu d'un filetage destiné à s'engager par couplage hélicoïdal dans le trou formé dans l'os, l'élément de fraisage (10) étant inséré coaxialement à travers l'élément tubulaire (30) de telle sorte que la partie terminale avant (11) de l'élément de fraisage (10) se trouve devant l'élément tubulaire (30), et pouvant tourner autour de l'axe longitudinal (A) par rapport à l'élément tubulaire (30) et pouvant effectuer un mouvement axial par rapport à l'élément tubulaire (30) ;
un moyen de limitation à une valeur prédéterminée du mouvement axial de l'élément de fraisage (10) par rapport à l'élément tubulaire (30),
et **en ce qu'**il comprend en outre un élément d'entraînement (40) agencé derrière l'élément tubulaire (30), fixé à l'élément de fraisage (10), qui fait tourner l'élément de fraisage (10) et le déplace axialement par rapport à l'élément tubulaire (30).

2. Instrument selon la revendication 1, **caractérisé en ce que** l'élément de fraisage (10) comporte une partie arrière (14) qui passe coaxialement à travers l'élément tubulaire (30) alors que la partie terminale avant (11) se projette vers l'avant de l'élément tubulaire (30).

3. Instrument selon la revendication 1, **caractérisé en ce que** l'élément d'entraînement (40) agit pour transmettre un couple à l'élément tubulaire (30).

4. Instrument selon la revendication 3, **caractérisé en ce que** 1"élément d'entraînement (40) peut librement glisser axialement et tourner par rapport à l'élément tubulaire (30) et est engagé par torsion avec celui-ci au moyen d'un engagement réciproque (35, 45) qui laisse à 1"élément d'entraînement (40) la liberté de pivoter d'un angle inférieur à 360 degrés et de se déplacer axialement par rapport à l'élément tubulaire (30).

5. Instrument selon la revendication 4, **caractérisé en ce que** le moyen d'engagement réciproque (35, 45) comprend des parties profilées relevées qui ressortent en direction axiale respectivement de l'élément tubulaire (30) et de 1"élément d'entraînement (40), qui sont conçus pour entrer en contact réciproque suite à une rotation réciproque de manière à transmettre un couple d'entraînement.

6. Instrument selon la revendication 5, **caractérisé en ce que** les parties profilées relevées (35, 45) sont agencées de manière à maintenir l'élément tubulaire (30) et 1"élément d'entraînement (40) à une distance axiale maximale prédéterminée quand ces éléments sont positionnés en contact torsionnel réciproque, et pour permettre l'approche des deux éléments à une distance axiale minimale par translation angulaire par rapport à la position de contact torsionnel réciproque.

7. Instrument selon la revendication 1, **caractérisé en ce qu'**il comprend un deuxième élément d'entraînement (50) fixé à l'élément tubulaire (30) et entraînant l'élément tubulaire (30) en rotation.

8. Instrument selon la revendication 7, **caractérisé en ce que** le deuxième élément d'entraînement (50) est coaxial avec le premier élément d'entraînement (40) et situé devant celui-ci.

9. Instrument selon la revendication 1, **caractérisé en ce qu'**il comprend des éléments de poussée agissant pour pousser axialement la tête de fraisage (11) vers l'avant par rapport à l'élément tubulaire (30).

10. Instrument selon la revendication 1, **caractérisé en ce qu'**il comprend un élément de sonde (20) de forme allongée situé à l'intérieur de l'élément de fraisage (10) et coaxialement par rapport à celui-ci, et pouvant glisser longitudinalement à travers l'élément de fraisage (10), son extrémité avant ressortant par rapport à l'extrémité avant de 1"élément de fraisage (10).

11. Instrument selon la revendication 10, caractérisé en qu'il comprend un moyen de signalisation du déplacement de l'élément de sonde (20) par rapport à l'élément de fraisage (10).

12. Instrument selon la revendication 10, **caractérisé en ce qu'**il comprend un moyen pour pousser axialement l'élément de sonde (20) de manière à forcer son extrémité avant à ressortir au-delà d'une extrémité avant de l'élément de fraisage (10).

13. Instrument selon la revendication 10, **caractérisé en ce qu'**il comprend un moyen pour ajuster une pression axiale appliquée à l'élément de sonde (20).

14. Instrument selon la revendication 10, **caractérisé en ce que** 1"élément de sonde (20) présente une partie arrière qui est visible par l'opérateur.

15. Instrument selon la revendication 10 comprenant une poignée de manipulation de l'instrument, **caractérisé en ce que** la partie arrière de l'élément de sonde (20) est conçue pour rester visible à l'arrière de la poignée.

16. Instrument selon la revendication 15, **caractérisé en ce que** l'élément de sonde (20) se déplace axialement du fait d'une pression appliquée à sa partie arrière, de manière à forcer son extrémité avant à ressortir d'une distance prédéfinie au-delà de la fin de l'extrémité avant de l'élément de fraisage (10).

17. Instrument selon la revendication 10, **caractérisé en ce que** 1"élément de sonde (20) présente des marquages millimétriques pour un contrôle et une détermination constants d'une position mesurée de la tête de fraisage (11) par rapport à la position de l'extrémité (72) du trou dans l'os.
